(19) Europäisches Patentamt
European Patent Office
Office européen des brevets

(11) **EP 3 520 758 B1**

(12) **EUROPÄISCHE PATENTSCHRIFT**

(45) Veröffentlichungstag und Bekanntmachung des Hinweises auf die Patenterteilung:
**18.11.2020 Patentblatt 2020/47**

(51) Int Cl.:
*A61F 13/537* (2006.01)   *D04H 1/4374* (2012.01)
*D04H 1/559* (2012.01)

(21) Anmeldenummer: **19152570.8**

(22) Anmeldetag: **18.01.2019**

(54) **FLÜSSIGKEITSAUFNAHME UND -VERTEILVLIES FÜR HYGIENEARTIKEL**

FLUID RETENTION AND DISTRIBUTION FABRIC FOR HYGIENE ARTICLES

NON-TISSÉ DE DISTRIBUTION ET DE RÉCEPTION DE LIQUIDE POUR ARTICLES D'HYGIÈNE

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **02.02.2018 DE 102018000854**

(43) Veröffentlichungstag der Anmeldung:
**07.08.2019 Patentblatt 2019/32**

(73) Patentinhaber: **Sandler AG**
**95126 Schwarzenbach/Saale (DE)**

(72) Erfinder:
• **Bernhuber, Uwe**
**95032 Hof (DE)**
• **Schuberth, Martin**
**95236 Stammbach (DE)**
• **Damke, Marco**
**95100 Selb (DE)**
• **Burger, Andreas**
**95100 Selb (DE)**

(74) Vertreter: **Grünecker Patent- und Rechtsanwälte PartG mbB**
**Leopoldstraße 4**
**80802 München (DE)**

(56) Entgegenhaltungen:
**EP-A1- 0 704 000      EP-A1- 3 238 680**
**EP-A2- 0 873 737      WO-A1-2017/095399**
**US-A- 4 883 707       US-A- 5 486 166**

**Beschreibung**

Wegwerfartikel für Hygieneanwendungen wie z.B. Damenbinden, Windeln sowie Inkontinenzprodukte haben den Zweck Körperflüssigkeiten so schnell wie möglich von der Haut wegzuleiten, um Hautirritationen zu vermeiden und dem Anwender ein sicheres und komfortables Gefühl zu geben. Für diesen Zweck sind Hygieneprodukte im Allgemeinen wie folgt aufgebaut:

- Erste Lage: Topsheet (Lochfolie oder Vlies)
- Zweite Lage: Acquisition and Distribution Layer, "ADL" (zumeist aus Vlies)
- Dritte Lage: Saugkern (bestehend aus Zellstoff und/oder Polyacrylsäure fixiert in einer oder mehreren Fixierlagen (SMS, ..)
- Abschlusslage: Folie, um ein Austreten der Flüssigkeit auf der Rückseite zu unterbinden

Die Lagen Topsheet, ADL und Saugkern müssen aufeinander abgestimmt sein, um eine Funktion im Hygieneprodukt zu gewährleisten.

Die wichtigste Rolle kommt dabei dem ADL als Transferlage zu. Dieser muss die Flüssigkeit vom Topsheet aufnehmen (Entwässerung des Topsheets), die Flüssigkeit in Längsrichtung des Produktes verteilen und kurz Zwischenspeichern. Durch das Zwischenspeichern soll sog. Gelblocking des Polyacrylsäure-Pulvers verhindert werden. Nach der kurzzeitigen Zwischenspeicherung wird die Flüssigkeit in den Saugkern zur finalen Speicherung weitergeleitet.

Um dieses Ziel zu erreichen kommen Aufnahme- und Verteillagen (kurz ADL aus engl. Acquisition Distribution Layer) aus Vliesstoffen zum Einsatz, die folgende Funktionen übernehmen sollen:

- Schnelle Aufnahme und Weiterleitung der Flüssigkeit
- Zwischenspeicherung zur Pufferung der begrenzten Aufnahmegeschwindigkeit des Absorbermaterials
- Gerichtete Verteilung der Flüssigkeit zur möglichst großflächigen Aufnahme durch das Absorbermaterial
- Kontrollierte Abgabe der Flüssigkeit an das Absorbermaterial um lokale Verblockung und Flüssigkeitsstau zu vermeiden.

Die wichtigsten Eigenschaften eines Aufnahme- und Verteilvlieses sind also Dicke, Dichte und die daraus resultierenden Porengrößen sowie die Kapillarität. Durch diese Eigenschaften wird das Aufnahme- und Verteilverhalten maßgeblich beeinflusst.

Hier kommen je nach Produkt Airlaid-, Highloft- und Spunlacevliese zur Anwendung. In der Anmeldung DE 10 2012 015 219 A1 sind verschiedene Ausführungen von Spunlace-Vliesen beschrieben, die entsprechende Eigenschaften aufweisen.

Die im Stand der Technik beschriebenen Vliesarten sind vornehmlich Einlagenaufbauten mit homogen verteilten Fasertypen. Abhängig von der Herstellungsart kann jeweils nur eine benötigte Eigenschaft erzielt werden. So bieten z.B. Highloft-Vliese, d.h. rein thermisch mittels Schmelzfasern verfestigte Vliesstoffe, durch ihre Struktur eine hohe Dicke und eine damit verbundene gute Flüssigkeitsaufnahme. Durch die vergleichsweise hohe Porosität ist aber die Verteilwirkung sehr gering. Dadurch ist nur eine stark begrenzte Nutzung des darunter liegenden Saugkerns möglich.

Die durch das Airlaid-Verfahren hergestellten Vliese haben durch die feinen Kapillaren eine gute Verteilwirkung. Durch den hohen Zellstoff-Anteil ist aber die Dicke niedrig und die Flüssigkeit wird wieder an die Oberfläche abgegeben, was einen hohen Rewet zur Folge hat.

Zweilagenaufbauten, die aus einem ein- bzw zweistufigen Herstellprozess stammen, wie beispielsweise in der DE102016005158, sind ebenfalls nicht zielführend. Die Verbindung der Schichten geschieht dabei mittels Wasserstrahlen, sodass bspw. Hydrophilierungsmittel abgewaschen werden, sodass die Flüssigkeitsaufnahme unzureichend ist. Desweiteren ist durch die Verfestigungsart die daraus resultierende Enddicke unzureichend, da speziell die ADL-Seite nur wenig Flüssigkeit aufnehmen kann.

Eine Verbindung der Lagen bei mehrlagigen Aufbauten mittels Kleber wie bspw mittels Hotmelt über Spraycoating oder Slotcoating resultiert zwar in einer ausreichenden Lagenhaftung, jedoch wird durch den Hotmelt die Flüssigkeitsweiterleitung erschwert. Es bilden sich an den Übergängen der Lagen mit Hotmelt blockierten Bereiche, die flüssigkeitsundurchlässig sind.

Materialien nach dem Stand der Technik nach dem Oberbegriff des Anspruchs 1 sind aus den Druckschriften DE 299 13 054 U1, US 5 531 727 A und EP 0 521 016 B1 bekannt. Sie kombinieren die Eigenschaften Flüssigkeitsverteilung und Flüssigkeitsaufnahme, wobei dennoch Abstriche in der jeweiligen Wirkung hingenommen werden müssen.

Die Aufgabe der vorliegenden Erfindung ist es daher, ein Material bereitzustellen, welches die genannten Nachteile des Standes der Technik vermeidet und eine verbesserte Flüssigkeitsaufnahme bei gleichzeitig gegebener Flüssigkeitsverteilung aufweist.

Gelöst wird die Aufgabe durch die im Anspruch 1 genannten Merkmale, sinnvolle Ausgestaltungen können den Ansprüchen 2 bis 3 entnommen werden.

Dies wird dadurch erreicht, dass der Vliesstoff in seinem vertikalen Aufbau, also senkrecht zur Lagenebene, wobei die Oberseite und die Unterseite mit unterschiedlicher Faserzusammensetzung und gegebenenfalls auch unterschiedlicher Verfestigungsart hergestellt wird. Dadurch kann jede Seite so ausgebildet werden, dass durch die Fasermischung und/oder die Verfestigungsart die jeweilige Funktion optimal erfüllt wird.

Der Verbund der Lagen geschieht erfindungsgemäß ausschließlich thermisch wobei die Fasermischung der Oberseite einen Anteil von mehr als 50Gewichtsprozent an Schmelzfasern aufweist. So ist es gewährleistet, eine ausreichende Anzahl von Bindepunkten der Oberseite mit der Unterseite auszubilden.

Durch den Übergang der Zonen wird zudem ein Gradient der physikalischen Eigenschaften erzeugt, so dass die Oberseite die größte Permeabilität und mechanische Stabilität aufweist, während die untere, dem Saugkern zugewandte Seite, die größte Kapillarität besitzt. Durch den Gradienten wird die Flüssigkeit schnell und nahezu restlos von der Körperseite weg und durch physikalische Kräfte erzwungen zum Saugkern transportiert.

Im Folgenden werden einige der in der folgenden Beschreibung verwendeten Begriffe näher definiert:
Textile Faserstoffe weisen zur Verbesserung der Verarbeitungseigenschaften aber auch zur Gewährleistung bestimmter Effekte im Fertigprodukt sogenannte "Avivagen" auf der Faseroberfläche auf.

Eine "Saugfaser" im Sinne der vorliegenden Erfindung ist eine regenerierte Zellulosefaser, welche aus Lösungen von Zellulosederivaten hergestellt wird. Diese Viskosefaser kann eine Modifikation der Oberfläche haben, z.B. trilobaler Querschnitt, aber auch insgesamt so modifiziert sein, dass eine moderate Flüssigkeitsaufnahme bei gleichzeitig guter Flüssigkeitsspeicherung begünstigt wird. Erfindungsgemäße Vertreter sind beispielsweise Viscostar-Viskosefasern oder normale Viskosefasern des Herstellers Lenzing, aber auch die sogenannte Galaxy-Faser der Fa Kehlheim Fibers. Im Besonderen können auch Viskosefasern welche nach dem Lyocell-Verfahren hergestellte werden (Tencel Fa. Lenzing) eingesetzt werden. Übliche Faserfeinheiten liegen im Bereich von 1,0 bis 3,3dtex, bevorzugt 1,3 bis 2,2 dtex. Kommen Stapelfasern zum Einsatz, liegen die eingesetzten Faserlängen im Bereich von 10 - 70mm, bevorzugt zwischen 35-50mm. Die Saugfasern weisen handelsübliche Kräuselungen auf. Der Begriff Saugfaser kann sich aber auch, sollte die Unterseite ein Airlaid darstellen, auf Kurzfasern oder Pulp beziehen.

Der Begriff "Matrixfaser" wird im Sinne der vorliegenden Erfindung für in der Oberseite enthaltene Stapelfaserstoffe aus thermoplastischen und/oder duroplastischen Polymeren verwendet. Bevorzugt sind Fasern aus thermoplastischen Polymeren wie Polyester oder Polypropylen. Die Schmelztemperatur muss dabei mindestens 10°C oberhalb der zum Einsatz kommenden Schmelzfasern liegen. Geeignet sind aber auch duroplastische Polymere wie beispielsweise Polyacrylfasern. Die Faserfeinheiten liegen im Bereich von 3,3 bis 17 dtex, bevorzugt 4,4 bis 10,0 dtex; die eingesetzten Faserlängen sind im Bereich von 10 - 80mm, bevorzugt 35-60mm. Die Verteilfasern weisen handelsübliche 2D oder auch 3D-Kräuselungen auf.

Die Begriffe "Avivierung" oder "Avivage" bezeichnend eine funktionelle Ausrüstung / Auflage auf Faseroberflächen. Diese Avivagen werden handelsüblich als Verarbeitungshilfen eingesetzt, d.h. Faser werden so behandeln, dass statischer Aufladung insbesondere bei PP oder PE-Fasern vorgebeugt wird. Zum Einsatz kommen Avivagen dabei in der Regel dabei auf die Oberfläche von Stapelfasern. Die Art der gewählten Avivagen können die Hydrophilie oder Hydrophobie beeinflussen, was großen Einfluss auf die Eigenschaften des Flüssigkeitsaufnahme- und -verteilvlies nehmen kann. Die Avivage sind sowohl auf den Oberflächen der Schmelzfasern, als auch auf den Matrixfasern und den Saugfasern vorhanden.

"Schmelzfasern" im Sinne der vorliegenden Erfindung sind Fasern, die in einem erfindungsgemäß aufgebauten Produkt

- den Verbund der Oberseite mit der Unterseite den Faserverbund ermöglichen
- die Struktur der Oberseite stabilisieren, sodass ein weitestgehend dimensionsstabiles Vlies entsteht.

Schmelzfasern sind homo- oder bikomponente, thermoplastische Polymerfaser, die schmelzbare Anteile aufweisen. Bei Beaufschlagung mit Temperatur schmelzen diese Anteile und stabilisieren nach dem Erkalten den Faserverbund. Erfindungsgemäß geeignete Fasern sind homopolymere Stapelfasern aus Co-Polyester, Co-Polyamid oder Polypropylen, erfindungsgemäß bevorzugt werden bikomponente Schmelzfasern in Kern-Mantel oder Side-by-Side Anordnung aus Kombinationen von niedrigschmelzendem Co-Polyester mit Polyester, Polyethylen mit Polypropylen oder Polyethylen mit Polyester. Die Faserfeinheiten für die in der Oberseite eingesetzten Schmelzfasern liegen im Bereich von 2,2 bis 12 dtex, bevorzugt 4,4 bis 6,7 dtex; die eingesetzten Faserlängen sind im Bereich von 10 - 80mm, bevorzugt 35-60mm.

Die Faserfeinheiten für die in der Unterseite eingesetzten Schmelzfasern liegen im Bereich von 1,7 bis 3.3 dtex, bevorzugt 1,7 bis 2,2 dtex; die eingesetzten Faserlängen sind, bei Verwendung von Stapelfasern im Bereich von 10 - 80mm, bevorzugt 35-60mm, bei Verwendung von Kurzfasern, sollte die Unterseite durch ein Airlaid-Vlies gebildet werden, zwischen 1 bis 5mm, bevorzugt 3mm. Sollten die Schmelzfasern kardierbare Stapelfasern sein, so weisen sie handelsübliche Kräuselungen auf.

Hergestellt werden erfindungsgemäße Vliesstoffe mittels Trocken- oder Naßverfahren, die zugrundeliegenden Verfahren können dem im Jahr 2000 bei Wiley VCH-Verlag, Weinheim erschienenen Buch "Vliesstoffe" entnommen werden. Speziell der die Unterseite bildende Vliesstoff kann ein luftgelegter Vliesstoff, ein sogenanntes "Airlaid" darstellen. Erfindungsgemäß bevorzugt werden kardierte Stapelfaservliesstoffe.

Die verwendeten Fasern werden bei der Erzeugung einer Fasermischung homogen miteinander vermischt. Die grundlegenden Techniken dazu sind ebenfalls im Buch "Vliesstoffe" aufgezeigt.

Als "Oberseite" wird erfindungsgemäß die Seite benannt, welche im Hygieneprodukt zum Benutzer hin gewandt ist und auf der Flüssigkeit zuerst auftrifft.

Die "Unterseite" ist die Seite des erfindungsgemäßen Vliesstoffes, die im Hygieneprodukt zum Saugkörper hin angebracht ist.

"Precursor" bezeichnet ein vorgefertigtes Vlies, welches bereits die Verfahrensschritte Fasermischung, Vliesbildung und Vliesverfestigung durchlaufen hat. Erfindungsgemäß wird die Unterseite durch einen Precursor gebildet.

Die nachfolgenden Beispiele beziehen sich, ohne darauf beschränkt zu sein, auf nach Trockenverfahren unter Verwendung des Kardierverfahrens hergestellte Stapelfaservliesstoffe.

Ergänzend dazu sind in Tabelle 1 die jeweils erzielten Prüfergebnisse aufgeführt. Die dabei ermittelten Parameter wurden gemäß den nachfolgend genannten Prüfmethoden ermittelt:

- Flächengewicht gemäß WSP 130.1., Angabe in kg/m$^2$
- Dicke gemäß WSP 120.6, Abschnitt 7.2, Messdruck 0,5kPa, Angabe in m
- Trennfestigkeit gemäß NWSP401,0.R0(15), Angabe in N
- Dicke der Oberseite wird gemäß folgender Formel ermittelt:

$$\text{Dicke Oberseite (m)} = \text{Gesamtdicke (m)} - \text{Dicke Precursor (m)}$$

- Dichte gemäß folgender Formel:

$$\text{Dichte (kg/m}^3\text{)} = \frac{\text{Flächengewicht (kg/m}^2\text{)}}{\text{Trockendicke (m)}}$$

- Strike Through gemäß WSP 70.3, Angabe in s
- Rewet gemäß WSP 80.10, Angabe in g
- Mittlerer Fasertiter innerhalb einer Lage: errechnet sich nach folgender Formel

$$\text{Mittlerer Fasertiter (dtex)} = \frac{A*\text{Titer 1} + B*\text{Titer 2} + C*\text{Titer 3}}{100}$$

A,B,C = Prozentanteil einer Faserkomponente in der Mischung, wobei die Summe aus A, B, und C = 100 ist.
Titer 1,2,3 = nominaler Titer der jeweiligen Faserkomponente in dtex

Die benötigten Eigenschaften, d.h. schnelle Flüssigkeitsaufnahme, gute Verteilung mit kurzzeitiger Zwischenspeicherung und Weiterleitung an den Saugkern werden erfindungsgemäß durch einen Aufbau aus zwei miteinander verbundenen Vlieslagen erzielt.

Ein erfindungsgemäß hergestelltes Flüssigkeitsaufnahme- und Verteilvlies durchläuft dabei folgende Verfahrensschritte:

- Herstellen einer Fasermischung für den Precursor, bestehend aus Saugfaser unter Zumischung von Schmelzfaser
- Herstellen einer unverfestigten Materialbahn aus der Fasermischung des Precursors
- Thermisches und/oder mechanisches Verfestigen der Materialbahn, sodass der Precursor mit einer Dichte von 50 bis 200 kg/m$^3$ und einer Dicke von 0,4 bis 1,0mm entsteht.
- Optional: Aufwickeln des Precursors

- Herstellen einer Fasermischung für die Oberseite, bestehend aus Matrixfaser und Schmelzfaser
- Herstellen einer unverfestigten Materialbahn aus der Fasermischung der Oberseite
- Ablegen der unverfestigten Materialbahn auf dem Precursor
- Verfestigung der Oberseite und Verbindung der Oberseite mit dem Precursor mittels einer Heißluft-Behandlung.
- Optional: Zusätzliche Passage eines Kalanders zur Stabilisierung des Verbundes. Die Verfestigungsfläche soll weniger als 10%, bevorzugt weniger als 7% betragen.
- Wickeln des erfindungsgemäßen Flüssigkeitsaufnahme- und Verteilvlieses

Der die Unterseite bildende Vliesstoff besteht dabei erfindungsgemäß aus einem Precursor, welcher einen Gehalt von 50 bis 80% Massenprozent an Saugfaser aufweist. Im Precursor werden Fasermischungen verwendet, die einen Unterschied des mittleren Fasertiters von Precursor zur Oberseite aufweisen. Erfindungsgemäß muss der Unterschied mindestens 2dtex betragen, wobei der mittlere Fasertiter der Fasermischung des Precursors geringer sein muss als der der Oberseite.

Der mittlere Fasertiter der Fasermischung des Precursors liegt im Bereich von 1,0 bis 6,0 dtex, wobei bevorzugt dieser kleiner 4dtex und am meisten bevorzugt kleiner 2,0dtex liegt.

Erfolgt die Herstellung des Precursors, wie erfindungsgemäß bevorzugt, als kardiertes Stapelfaservlies, ergibt sich eine Faserorientierung in Fertigungsrichtung des Vliesstoffes. Diese Orientierung ist für die spätere Verwendung vorteilhaft, da die Faserorientierung dann ebenfalls in Längsrichtung des fertigen Hygieneproduktes verläuft. Dadurch wird die Ausnutzung der zur Verfügung stehenden Saugfläche im Hygieneprodukt verbessert.

Bei der Verfestigung des Precursors, welche erfindungsgemäß bevorzugt, aber ohne darauf beschränkt zu sein, mittels Wasserstrahlen geschieht, erfolgt gleichzeitig eine Verdichtung des Precursors, sodass die Dichte des Precursors höher liegt als die des letztendlichen Aufnahme- und Verteilvliesstoffes. Vorgesehen werden dabei Dichten des Precursors von 50 bis 200kg/m$^3$, bevorzugt 80 bis 120kg/m$^3$, wobei der Dickenbereich des Precursors zwischen 0,4 bis 1mm liegt und ein Flächengewicht von 0,025 bis 0,120 kg/m2 angestrebt wird.

Aufgrund des gewählten mittleren Fasertiters des Precursors in Verbindung mit der Verdichtung während der Verfestigung des Precursors wird die Kapillarwirkung und damit die Fähigkeit innerhalb des Precursors Flüssigkeit zu transportieren, positiv beeinflusst.

Erfindungsgemäß wird nun auf diesen Precursor ein unverfestigter, kardierter Faserflor abgelegt. Dieser Faserflor bildet nach einem daran anschließenden Verfestigungs- und Verbindungsschritt die Oberseite des erfindungsgemäßen Vliesstoffs.

Die Verfestigung des Faserflors der Oberseite und der Verbund von der Oberseite mit dem Precursor geschieht erfindungsgemäß mittels thermischer Behandlung, d.h mittels Heißluftverfestigung und einer optional anschließenden Kalanderverfestigung. Die grundlegenden Techniken können dabei dem Buch "Vliesstoffe", erschienen bei Wiley-VCH im Jahr 2012 den Seiten 375-395 entnommen werden.

Der Faserflor der Oberseite besteht dabei zu 50 bis 0 Gewichtsprozent aus Matrixfasern und zu 50 bis 100 Massenprozent aus Schmelzfasern.

Erfindungsgemäß notwendig ist in der Fasermischung, welche die Oberseite bildet, ein Anteil von mehr als 50% Schmelzfasern. Dieser Anteil ist im Vergleich zum Stand der Technik wesentlich und gewährleistet eine ausreichende Anzahl von möglichen Bindepunkten zwischen der Oberseite und der Unterseite.

Legt man den unverfestigten Faserflor, welcher nach Verfestigung die Oberseite bildet, auf die Unterseite, ergeben sich an der Grenzschicht zwischen Ober- und Unterseite nur wenig Berührungspunkte, die nach Heißluftbehandlung verschmelzen können bzw Bindepunkte ausbilden können.

Notwendig und erfindungsgemäß bevorzugt, allerdings ohne darauf beschränkt zu sein, ist es daher für die Heißluftverfestigung eine Anlage vorzusehen, die nach dem Durchströmungsprinzip arbeitet. Dabei trifft heiße Luft auf den zunächst unverfestigten Faserflor der Oberseite und durchströmt die Oberseite gefolgt von der Unterseite. In Abhängigkeit von der Menge der durchströmenden Heißluft und der Durchströmungsgeschwindigkeit erfährt der Faserflor der Oberseite ein geringere oder größere Verdichtung im Vergleich zur Dicke des unverfestigten Faserflores.

Mittels dieser Verdichtung bildet sich auf der Grenzschicht Oberseite zu Unterseite eine deutlich größere Anzahl von Kontaktstellen und daher möglichen Bindepunkten von Schmelzfasern im Vergleich zum einfachen "Auflegen" des Faserflores und Verfestigung mittels Bedüsung oder Strahlungswärme.

Zur Gewährleistung der Haftung der Oberseite mit der Unterseite werden Schmelzfasern in der Fasermischung der Unterseite eingesetzt. Dies da die Verbindung zwischen Schmelzfasern erleichtert wird und derartige Bindepunkte von Schmelzfasern aus der Oberseite mit der Unterseite mechanisch beständiger sind.

Besonders vorteilhaft hat sich die Heißluft-Verfestigung und -Verbindung in Bezug auf die Avivierung der Fasern der Oberseite gezeigt. Wird ein Verbund mittels Wasserstrahlverwirbelung erzeugt, wird die auf den Fasern vorhandene Avivage durch die Wasserstrahlen fast vollständig abgewaschen. Mittels Avivage erzielte Faser- oder Vlieseigenschaften werden dadurch verändert, sodass Anforderungsprofile nicht erfüllt werden können. Durch die Heißluft-Verfestigung und -Verbindung bleiben die Avivagen auf den Faseroberflächen erhalten und die Eigenschaften werden nicht beeinflusst.

Für den Precursor ist die Obergrenze für den Gehalt von Schmelzfaser bei 50% Gewichtsprozent, da mit Anteilen größer 50 Gewichtsprozent% die Flüssigkeitsspeicherung leidet und andererseits der Precursor zu steif wird.

Erfindungsgemäß bevorzugt können auch gleichartige Schmelzfasern, d.h. aus gleichen Polymeren eingesetzt werden, sodass die Haftung von Oberseite zu Unterseite verbessert wird.

In einer anderen bevorzugten Ausführung, insbesondere für den Fall, dass im Precursor ausschließlich Saugfasern enthalten sind, kann eine Schmelzfasertype eingesetzt werden, die eine besondere Affinität zu zellulosischen Polymeren aufweist.

Der mittlere Titer der Fasermischung der Oberseite liegt größer 3,3dtex, bevorzugt im Bereich von 4,4 bis 12 dtex und besonders bevorzugt im Bereich von 6,7 bis 12dtex. Durch diesen mittleren Fasertiter ist das die Oberseite bildende Vlies offen genug um Flüssigkeit rasch aufzunehmen und bis zur Weiterleitung an die Unterseite zwischen zu speichern. Je höher der mittlere Faserdurchmesser der Mischung ist, umso besser ist auch die Beständigkeit der Oberseite gegenüber Druckbelastungen. Mit ansteigenden Anteilen von Schmelzfasern wird die Oberseite auch mechanisch stabiler, d.h. hat eine verbesserte Resistenz gegen Kompression wie beispielweise hohe Packungsdichte von Windeln oder gegen punktuelle Belastungen im Gebrauch.

Der Rewet wird mit höheren mittleren Fasertitern und mit Schmelzfaseranteil größer 75 Gewichtprozent positiv beeinflusst.

Durch eine thermische Aktivierung der Schmelzfasern wird insbesondere die Stabilität der Oberseite gewährleistet. Dadurch ist eine verbesserte Widerstandsfähigkeit gegenüber die bereits vorerwähnte Kompression in der Produktanwendung gewährleistet.

Erfindungsgemäß werden die Verfestigungsbedingungen in Kombination mit der Fasermischung der Oberseite so gewählt, dass das erfindungsgemäße Flüssigkeitsaufnahme- und Verteilvlies eine Dicke von größer 3mm, bevorzugt größer 5mm aufweist.

Für den Fall, dass in der Fasermischung, welche die Unterseite bildet, der Anteil von Schmelzfaser kleiner 30 Gewichtsprozent% liegt, kann eine optionale thermische Kalander-Behandlung zur Verbesserung der Haftung von Oberseite zur Unterseite vorgesehen werden. Die Haftung wird über die Trennfestigkeit ermittelt.

Um die Dicke des Flüssigkeitsaufnahme- und Verteilvlieses nicht zu beeinflussen, ist es daher die Anordnung des Kalanders und die Kalandergravur entsprechend zu wählen.

Die Gravurwalze muss so angeordnet sein, dass die Prägung auf der Oberseite entsteht. Die gravierte Walze muss einerseits eine Pressfläche von kleiner 10%, bevorzugt kleiner 7% haben, anderseits die Steghöhe, d.h. die Distanz von Gravurgrund zu Pressplateau, größer 2mm betragen. Erfindungsgemäß werden Gravuren eingesetzt, die, sofern nicht als Linien ausgebildet, weniger als 10 Gravurpunkte pro cm$^2$ aufweisen, besonders bevorzugt sind Gravuren mit 7 oder weniger Gravurpunkte pro cm$^2$.

Das erfindungsgemäße Flüssigkeitsaufnahme- und Verteilvlieses hat zur Gewährleistung der Flüssigkeitsaufnahme in der Oberseite eine offene, mechanisch stabile Vliesstruktur und zur Verteilung eine verdichte Unterseite um Flüssigkeiten aufzusaugen und aufgrund Kapillarität zu verteilen.

Die Anforderungen an die Oberseite werden dabei erreicht durch:

- Mittleren Titer der Fasermischung > 4,0 dtex
- Dicke der Oberseite > 0,002m
- Gewicht der Oberseite > 0,04kg/m$^2$

Die Anforderungen an die Unterseite werden dabei erreicht durch:

- Mittleren Titer der Fasermischung <6,0dtex
- Dicke der Unterseite < 0,001m
- Gewicht der Unterseite < 0,06kg/m$^2$

Die Anforderungen an die Verbindung von Oberseite mit Unterseite, wobei die Dicke der Oberseite nicht wesentlich beeinflusst wird, werden erreicht durch die Bereitstellung von

- Anteil der Schmelzfasern > 50 Gewichtsprozent in der Fasermischung der Oberseite
- Anteil der Schmelzfasern von 20- 50 Gewichtsprozent in der Fasermischung der Unterseite
- Anwendung von Heißluftbehandlung mit Luftströmung von Oberseite durch Unterseite.
- Optionale Anwendung einer nach der Heißluftbehandlung sich anschließenden Kalanderbehandlung.

In der Kombination der Merkmale der Oberseite mit der Unterseite ergibt sich, dass ein erfindungsgemäß ausgeführtes Flüssigkeitsaufnahme- und Verteilvlies folgende Merkmale aufweist:

- Die Dicke der Oberseite hat einen Anteil von mindestens 60% der Gesamtdicke

- Die Dichte der Unterseite ist mindestens doppelt so hoch wie die Dichte der Oberseite

- Der mittlere Fasertiter der Oberseite muss mindestens 2,0dtex größer sein als der mittlere Fasertiter der Unterseite.

- Die Dichte des Flüssigkeitsaufnahme- und Verteilvlies beträgt maximal 35kg/m$^3$

Betrachtet man Tabelle 1 so kann man folgendes erkennen:
Das Muster 1 repräsentiert ein gemäß der DE102016005158 hergestelltes, dem Stand der Technik zurechenbares Flüssigkeitsaufnahme- und Verteilvlies. Der Verbund der Oberseite mit der Unterseite geschieht mittels Wasserstrahlen. Obwohl einen mittleren Fasertiter von 6.2dtex der Oberseite und einen mittleren Fasertiter der Unterseite von 2,2 dtex, liegt die Dicke durch die Komprimierung der Wasserstrahlen bei 0,00178m. Der Strike-Through beträgt 9.4s.

Die Muster 2 bis 5 wurden mittels einer Heißluft-Verfestigung in einem Bandtrockner behandelt. Für die Unterseite wurde dabei ein bereits mittels Wasserstrahlen verfestigtes Vlies als Precursor verwendet. Auf dieses, die Unterseite bildende Vlies wurde dann erfindungsgemäß der unverfestigte Faserflor aufgelegt und der Faserflor verfestigt, sodass sich die Oberseite gebildet hat. Der Verbund Oberseite zu Unterseite geschah mittels der gerichteter Luftführung der Heißluft von der Oberseite in Richtung der Unterseite. Die zur Anwendung kommenden Trocknertemperaturen richten sich nach der Art der Polymere und Schmelzviskosität. Bei den Mustern 2 bis 5 kamen Temperaturen, ohne darauf beschränkt zu sein, im Bereich von 120 bis 140°C zum Einsatz.

Betrachtet man das erfindungsgemäß ausgeführte Muster 2, welches mit identischen Fasermischungen der Oberseite gemäß Muster 1 hergestellt wurde, wobei die Verbindung von Oberseite mit Unterseite erfindungsgemäßen mittels Heißluft an einem Flachbandtrockner geschah, erkennt man, dass die Gesamtdicke um den Faktor 3 höher im Vergleich zum Muster 1 liegt. Durch die geringere Komprimierung der Oberseite und auch das Vorhandensein von hydrophiler Avivage auf allen Fasern der Oberseite ergibt sich ein im Vergleich zum Stand der Technik verbesserter Strike Through. Die Trennfestigkeit liegt auf einem akzeptablen Niveau, d.h. bei der Verarbeitung bleibt die Verbindung von Oberseite zu Unterseite erhalten. Der Unterschied des mittleren Fasertiters von Ober- zu Unterseite beträgt 4,5 dtex. Das Muster 1 hat den gleichen Unterschied des Titers, allerdings hat die Oberseite eine deutlich geringere Dicke im Vergleich zur Oberseite des Musters 2.

Durch die Verwendung von Matrixfasern mit sehr hohen Titer, d.h. 10dtex, wird die hohe Dicke der Oberseite des Musters 2 gewährleistet. Dadurch ist ein sehr hohe Porosität gewährleistet. Die Dichte des erfindungsgemäßen Musters 2 bei 17kg/m$^3$, diese ist im Vergleich zu Muster 1 um 3,8 geringer.

Bei dem erfindungsgemäß hergestellten Muster 3 wird auf den Einsatz von Matrixfasern verzichtet. Der Anteil von Schmelzfasern liegt bei 100Gewichtsprozent. Durch den Unterschieds des mittleren Fasertiters der Mischung Oberseite zu Unterseite von 3.1dtex ergibt sich wieder die Porosität der Oberseite. Die Trennfestigkeit liegt aufgrund des Schmelzfaser-Anteils von 100Gewichtsprozent auf einem höheren Level im Vergleich zu Muster 2. Der Unterschied des mittleren Fasertiters Oberseite zu Unterseite beträgt bei Muster 3 3,1dtex. StrikeThrough und auch Rewet sind in einem Bereich, der Muster 2 entspricht. Die Dichte bei 29kg/m$^3$, was im Vergleich zu Muster 2 knapp doppelt so hoch ist. Diese höhere Dichte bedingt einen etwas höheren Intake.

Muster 4 sieht im Vergleich zu den Mustern 2 und 3 die Verwendung von lediglich 20 Gewichtsprozent Schmelzfasern in der Unterseite vor. Die Lagenhaftung ist daher deutlich geringer im Vergleich zu Muster 2 und 3, sodass dieser Anteil von Schmelzfasern in der Unterseite die Untergrenze definiert.

Im Muster 5 wurde eine der Heißluftverfestigung nachfolgende Kalanderbehandlung durchgeführt. Dabei wurde eine Kalandergravur benutzt, die bei einer Steghöhe von 2mm eine Pressfläche von 5% und 9 Figuren / cm$^2$ aufweist. Der Druck betrug 75daN/cm, die Walzentemperatur 125°C. Die Gravurwalze drückt dabei auf die Oberseite.

Neben einem positiven Einfluss auf die Lagenhaftung ist auch eine leichte Erhöhung der Kapillarwirkung an der Oberfläche der Oberseite gegeben. Dies begünstigt eine gerichtete Flüssigkeitsweiterleitung.

Betrachtet man das Muster 5, wobei der mittlere Fasertiter der Oberseite bei 4,4 dtex liegt, zeigt sich im Vergleich zu den erfindungsgemäß ausgeführten Mustern 2 bis 4 eine höhere Dichte, jedoch liegt der StrikeThrough und auch der Rewet im Bereich der Musters 2 bis 4.

In weiteren erfindungsgemäßen Ausführungen können auch Kalandergravuren in Form von Linien ähnlich der DE10103627 zum Einsatz kommen. Auch hier ist zu beachten, dass die Steghöhe von 2mm und höher ist und auch die Pressfläche kleiner 10% beträgt.

**Tabelle 1**

| | Lagen | Mischung | Dicke [m] | | Dichte Gesamt [kg/m³] | Mittlerer Fasertiter [dtex] | Flächengewicht [kg/m²] | | Strike Through [sec] | Rewet [g] |
|---|---|---|---|---|---|---|---|---|---|---|
| Muster 1 | Oberseite | 30% Matrixfaser PET 10dtex / 50% Schmelzfaser Bikomponent PE(Mantel) /PET (Kern) 5,8 dtex/ 20% Matrixfaser PET 1,7 dtex | 0,0013 | Gesamt: 0,0018 | 67 | 6,2 | 0,085 | Gesamt 0,12 | 9,4 | 0,09 |
| | Unterseite | 100% Saugfaser CV 1,7 dtex | 0,0005 | | | 1,7 | 0,035 | | | |
| | | | | | | | | | | |
| Muster 2 | Oberseite | 30% Matrixfaser PET 1 0dtex / 50% Schmelzfaser Bikomponent PE(Mantel) /PET (Kern) 5,8 dtex / 20% Matrixfaser PET 1,7 dtex | 0,0063 | Gesamt: 0,0068 | 18 | 6,2 | 0,085 | Gesamt 0,12 | 0,42 | 0,45 |
| | Unterseite | 70% Saugfaser CV 1,7 dtex / 30% Schmelzfaser Bikomponent PE (Mantel) /PP (Kern), 1,7dtex | 0,0005 | | | 1,7 | 0,035 | | | |
| | | | | | | | | | | |
| Muster 3 | Oberseite | 60% Schmelzfaser Bikomponent PE (Mantel) /PP (Kern) 6,7dtex / 40% Schmelzfaser Bikomponent PE (Mantel) /PP (Kern) 1,7dtex | 0,0057 | Gesamt: 0,0062 | 29 | 4,7 | 0,12 | Gesamt 0,18 | 0,46 | 0,68 |
| | Unterseite | 70% Saugfaser CV 1,7 dtex / 30% Schmelzfaser Bikomponent PE (Mantel) /PP (Kern), 1,7dtex | 0,0005 | | | 1,7 | 0,06 | | | |
| | | | | | | | | | | |
| Muster 4 | Oberseite | 60% Schmelzfaser Bikomponent PE (Mantel) /PP (Kern) 6,7dtex / 40% Schmelzfaser Bikomponent PE (Mantel) /PP (Kern) 1,7dtex | 0,0055 | Gesamt: 0,0059 | 24 | 4,7 | 0,1 | Gesamt 0,14 | 0,8 | 0,07 |
| | Unterseite | 80% Saugfaser CV 1,7 dtex / 20% SchmelzfaserBikomponent PE (Mantel) /PP (Kern) 1,3 dtex | 0,0004 | | | 1,6 | 0,04 | | | |
| | | | | | | | | | | |

(fortgesetzt)

| | Lagen | Mischung | Dicke [m] | | Dichte Gesamt [kg/m³] | Mittlerer Fasertiter [dtex] | Flächengewicht [kg/m²] | | Strike Through [sec] | Rewet [g] |
|---|---|---|---|---|---|---|---|---|---|---|
| Muster 5 | Oberseite | 100% Schmelzfaser Bikomponent PE (Mantel) /PET (Kern) 4,4 dtex | 0,0038 | Gesamt: 0,0043 | 30 | 4,4 | 0,07 | Gesamt 0,13 | 0,8 | 0,52 |
| | Unterseite | 80% Saugfaser CV 1,7 dtex / 20% Schmelzfaser Bikomponent PE (Mantel) /PP (Kern) 1,3 dtex | 0,0005 | | | 1,6 | 0,06 | | | |

**EP 3 520 758 B1**

**Patentansprüche**

1. Mehrlagiges Flüssigkeitsaufnahme und -verteilvlies für Hygieneprodukte welches eine Oberseite, welche in Hygieneprodukten zum Benutzer hin gewandt ist, und eine Unterseite, welche in Hygieneprodukten zum Saugkörper hin angebracht ist, aufweist, wobei die Unterseite aus einem Vliesstoff besteht, dessen Fasermischung aus 50 bis 80 Gewichtsprozent Saugfasern und 50 bis 20 Gewichtsprozent Schmelzfasern besteht und die Oberseite aus einem Vliesstoff besteht, dessen Fasermischung aus 50 bis 100 Gewichtsprozent Schmelzfasern und 50 bis 0 Gewichtsprozent Matrixfasern besteht, die Fasermischung des die Unterseite bildenden Vliesstoffs einen geringeren mittleren Fasertiter aufweist als die Fasermischung des die Oberseite bildenden Vliesstoffs, wobei die Oberseite mit der Unterseite ausschließlich thermisch schmelzverbunden ist und das Flüssigkeitsaufnahme und -verteilvlies eine Dichte von 35kg/m$^3$ oder weniger beträgt,
**dadurch gekennzeichnet, dass**

   - die Oberseite einen mittleren Titer der Fasermischung > 4,0 dtex, eine Dicke > 0,002m und ein Gewicht> 0,04kg/m$^2$ aufweist,
   - die Unterseite einen mittleren Titer der Fasermischung < 6,0dtex, eine Dicke < 0,001m und ein Gewicht < 0,06 kg/m2 aufweist,
   - die Dicke der Oberseite einen Anteil von mindestens 60% der Gesamtdicke aufweist,
   - die Dichte der Unterseite mindestens doppelt so hoch ist wie die Dichte der Oberseite
   - der mittlere Fasertiter der Fasermischung des die Unterseite bildenden Vliesstoffs mindestens 2dtex geringer ist als der mittlere Fasertiter der Fasermischung des die Oberseite bildenden Vliesstoffs.

2. Mehrlagiges Flüssigkeitsaufnahme und -verteilvlies nach Anspruch 1,
**dadurch gekennzeichnet, dass**

   - die Oberseite mit der Unterseite durch eine Heißluftbehandlung thermisch schmelzverbunden ist oder
   - die Oberseite mit der Unterseite durch eine Heißluftbehandlung und mit einer nachgeschalteten Kalanderbehandlung thermisch schmelzverbunden ist.

3. Mehrlagiges Flüssigkeitsaufnahme und -verteilvlies nach einem der vorangehenden Ansprüche,
**dadurch gekennzeichnet, dass**

   - die Schmelzkomponente der Schmelzfasern in der Oberseite und der Unterseite aus gleichen Polymeren bestehen.

**Claims**

1. Multi-layer nonwoven material for absorbing and distributing liquids for hygiene articles which has a top layer which in hygiene articles is facing towards the user and a bottom layer which in hygiene articles is attached facing the absorbent body, the bottom layer consisting of a nonwoven fabric the fibre mixture of which consists of 50 to 80% by weight of absorbent fibres and of 50 to 20% by weight of fusible fibres, and the top layer consisting of a nonwoven fabric whose fibre mixture consists of 50 to 100% by weight of fusible fibres and 50 to 0% by weight of matrix fibres, the fibre mixture of the nonwoven fabric forming the bottom layer has an average fibre count lower than the fibre mixture of the nonwoven forming the top layer, wherein the top layer is exclusively thermally fused to the bottom layer and the nonwoven material for absorbing and distributing liquids has a density of 35kg/m$^3$ or less,
**characterised in that**

   - the top layer has an average count of the fibre blend > 4,0 dtex, a thickness > 0,002 m and a weight > 0,04 kg/m$^2$,
   - the bottom layer has an average count of the fibre blend > 6 dtex, a thickness > 0,001 m and a weight > 0,06 kg/m$^2$,
   - the thickness of the top layer accounts for at least 60% of the total thickness,
   - the density of the bottom layer is at least double the density of the top layer,
   - the average fibre count of the fibre mixture of the non-woven fabric forming the bottom layer is at least 2 dtex smaller than the average fibre count of the fibre mixture of the non-woven fabric forming the top layer.

2. Multi-layer nonwoven material for absorbing and distributing liquids according to claim 1,
**characterised in that**

- the top layer is thermally fused to the bottom layer by a hot air process, or
- the top layer is thermally fused to the bottom layer by a hot air process and a subsequent calendering process.

3. Multi-layer nonwoven material for absorbing and distributing liquids according to any of the preceding claims, **characterised in that**

- the fusible components of the fusible fibres in the top layer and in the bottom layer are made of the same polymers.

**Revendications**

1. Non-tissé multicouche d'absorption et de répartition de liquide pour produits d'hygiène, présentant une face supérieure tournée, au sein des produits d'hygiène, vers l'utilisateur et une face inférieure appliquée, au sein des produits d'hygiène, contre le corps absorbant, dans lequel la face inférieure est constituée d'un tissu non-tissé dont le mélange de fibres est constitué de 50 à 80 pour cent en poids de fibres absorbantes et de 50 à 20 pour cent en poids de fibres fondues et la face supérieure est constituée d'un tissu non-tissé dont le mélange de fibres est constitué de 50 à 100 pour cent en poids de fibres fondues et de 50 à 0 pour cent en poids de fibres de matrice, le mélange de fibres du tissu non-tissé formant la face inférieure présente un titre moyen de fibres inférieur à celui du mélange de fibres du tissu non-tissé formant la face supérieure, dans lequel la face supérieure est liée par fusion de manière exclusivement thermique à la face inférieure et le non-tissé d'absorption et de répartition de liquide présente une densité inférieure ou égale à 35 kg/m$^3$, **caractérisé en ce que**

- la face supérieure présente un titre moyen du mélange de fibres > 4,0 dtex, une épaisseur > 0,002 m et un poids > 0,04 kg/m$^2$,
- la face inférieure présente un titre moyen du mélange de fibres < 6,0 dtex, une épaisseur < 0,001m et un poids < 0,06 kg/m$^2$,
- l'épaisseur de la face supérieure représente au moins 60 % de l'épaisseur totale,
- la densité de la face inférieure est d'au moins le double de la densité de la face supérieure
- le titre de fibre moyen du mélange de fibres du tissu non-tissé formant la face inférieure est inférieur d'au moins 2 dtex au titre de fibre moyen du mélange de fibres du tissu non-tissé formant la face supérieure.

2. Non-tissé multicouche d'absorption et de répartition de liquide selon la revendication 1, **caractérisé en ce que**

- la face supérieure est liée par fusion de manière thermique à la face inférieure grâce à un traitement par air chaud ou
- la face supérieure est liée par fusion de manière thermique à la face inférieure grâce à un traitement par air chaud suivi d'un traitement par calandrage.

3. Non-tissé multicouche d'absorption et de répartition de liquide selon l'une quelconque des revendications précédentes, **caractérisé en ce que**

- les composants fondus des fibres fondues au sein de la face supérieure et de la face inférieure sont constitués des mêmes polymères.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102012015219 A1
- DE 102016005158
- DE 29913054 U1
- US 5531727 A
- EP 0521016 B1
- DE 10103627

**In der Beschreibung aufgeführte Nicht-Patentliteratur**

- Vliesstoffe. Wiley VCH-Verlag, 2000
- Vliesstoffe. Wiley-VCH, 2012, 375-395